# EUROPÄISCHE PATENTSCHRIFT

(11) **EP 2 224 910 B1**
(45) Veröffentlichungstag und Bekanntmachung des Hinweises auf die Patenterteilung: **12.03.2014**
(21) Anmeldenummer: 08852010.1
(22) Anmeldetag: 19.11.2008
(51) Int. Cl.: A61K 9/08, A61K 47/06

(54) **SPÜLLÖSUNG**
RINSE SOLUTION
SOLUTION DE RINÇAGE

(30) Priorität: 19.11.2007 DE 102007055046
(43) Veröffentlichungstag der Anmeldung: 08.09.2010
(73) Patentinhaber: FLUORON GMBH, 89077 Ulm (DE)
(72) Erfinder: WONG, David, Merseyside CH63 6HT (GB); LINGENFELDER, Christian, 89081 Ulm (DE)
(74) Vertreter: Schiweck, Weinzierl & Koch
(86) Internationale Anmeldenummer: PCT/EP2008/009769
(87) Internationale Veröffentlichungsnummer: WO 2009/065565

(56) Entgegenhaltungen:
- DE-A1-102005 017 279
- US-A- 5 219 844
- US-A- 5 336 487
- US-A1- 2006 177 524
- US-B1- 6 290 690

## Beschreibung

### Gebiet der Erfindung

Die Erfindung betrifft ein Silikonöl mit einer Dichte von weniger als 1 g/cm³ und einer Viskosität von nicht mehr als 100 mPas, gemessen mit einem Ubbelohde-Kapillarviskosimeter bei einer Temperatur von 25°C bei Umgebungsdruck, zur Verwendung in einem Verfahren bei ophthalmologischen Eingriffen, umfassend Verwenden des Silikonöls als Spüllösung bei dem ophthalmologischen Eingriff.

### Hintergrund der Erfindung

Während chirurgischer Eingriffe ist es häufig notwendig während des Eingriffs zu spülen, um das Operationsfeld von unerwünschtem Material zu befreien. Hierzu wird in der Regel isotonische Kochsalzlösung, gegebenenfalls mit Zusätzen, verwendet.

Viele Erkrankungen der Augen von Wirbeltieren sind auf Veränderungen der Netzhaut oder des Glaskörpers zurückzuführen. Der Glaskörper ist ein Bestandteil der Augen und dient zur Erhaltung der spezifischen Form des Auges. Der Glaskörper ist dabei zwischen der Netzhaut des Auges und der Linse angeordnet und besteht aus ca. 98 % Wasser und ca. 2 % Hyaluronsäure und einem Netz von Kollagenfasern, die zur Stabilisierung des Wassers aufgrund ihres Retentionsvermögens dienen. Dadurch entsteht die gelartige Konsistenz und Durchsichtigkeit des Glaskörpers.

Die Möglichkeiten zur Behandlung von Erkrankungen am Auge, insbesondere der Netzhaut, wie Netzhautablösung, haben in den letzten Jahren stark zugenommen. Für den Arzt, der diese Behandlung durchführt, ist es wichtig, die erkrankten oder geschädigten Bereiche gut zu erkennen. Voraussetzung für erfolgreiche chirurgische Eingriffe allgemein und insbesondere am Auge ist die gute Sicht auf die anatomischen Strukturen.

Teil einer derartigen Behandlung kann die operative Entfernung des Glaskörpers (Vitrektomie) sein. Zur Entfernung des Glaskörpers werden drei kleine Schnitte in der Sklera des Auges vorgenommen, die in die hintere Augenkammer reichen, in der sich der Glaskörper befindet. Durch die Einschnitte können dann entsprechende Operationsinstrumente in das Innere des Auges eingeführt werden. Um das Auge sowenig wie möglich zu beschädigen, sollten die Einschnitte sehr klein sein. Während des Eingriffs wird das Operationsfeld gespült, in der Regel mit Kochsalzlösung.

Damit das Auge aufgrund des Druckverlustes beim Absaugen des die Stabilität bildenden Glaskörpers nicht die Form verliert und Sekundärschäden davonträgt, wird an Stelle des Glaskörpers eine Ersatzflüssigkeit in den Hohlraum eingeleitet, die den Innendruck konstant hält. Im Stand der Technik verwendet man als Spüllösung üblicherweise eine isotonische Kochsalzlösung wegen ihrer chemischen Zusammensetzung und ihrer physikalischen Eigenschaften, da eine Kochsalzlösung optimal mit dem Wasser des Glaskörpers mischbar ist, so dass ein Austausch der beiden Flüssigkeiten problemlos erfolgen kann.

Während der Behandlung ist es notwendig, den Ort des chirurgischen Eingriffs zu spülen. Dabei darf aber die Sicht auf das Operationsfeld nicht eingeschränkt werden. Außerdem muss die Spüllösung physiologisch verträglich sein, sie muss gut einführbar und leicht entfernbar sein.

Es wurde nun gefunden, dass die häufig verwendete isotonische Kochsalzlösung aufgrund ihrer guten Mischbarkeit mit Wasser den Nachteil hat, dass sie sich mit den anderen Flüssigkeiten, wie Kammerwasser und Blut, vermischt und dadurch eingefärbt und eingetrübt wird. Diese Eintrübung fuhrt zu einer Einschränkung der Sicht auf das Operationsfeld, was äußerst unerwünscht ist.

Weiterhin werden standardmäßig der Kochsalzlösung Glutathion und Glucose zugesetzt. Da eine derartige Lösung instabil ist, kann Gluthathion und Glucose allerdings erst direkt vor der Verwendung zugesetzt werden, was aufwändig ist und die Kontaminationsgefahr erhöht.

Obwohl es im Stand der Technik Anregungen zur Verwendung verschiedenartigster Tamponadeflüssigkeiten, siehe z.B. DE 10 2005 017279, US 5,219,844, US 5,366,487 oder US 6,290,690 gibt, werden für die während des chirurgischen Eingriffs eingesetzten Spüllösungen üblicherweise nur wässrige Lösungen, die bevorzugt in etwa die Dichte des Wassers haben, genannt.

So beschreibt DE 60107451T2 die Verwendung einer Kochsalzlösung als herkömmlichem ophthalmischen Fluid zum Spülen während einer Vitrektomie. Nach der Vitrektomie ist es notwendig, eine den Glaskörper (vorübergehend) ersetzende Substanz einzubringen. In diesem Zusammenhang beschreibt WO 03/079927 die Verwendung von perfluorierten Alkanen als Austauschflüssigkeit und Tamponadepräparat bei chirurgischen Eingriffen, insbesondere an der Netzhaut. Derartige perfluorierte Alkane haben den Vorteil, sich aufgrund ihrer hohen Dichte besonders im unteren Teil des Glaskörpers anzusammeln und somit die Behandlung für den Chirurgen an der Netzhaut zu erleichtern. Diese hohe Dichte ist aber auch von Nachteil, da die perfluorierten Alkane aufgrund der hohen Dichte Druck auf die Gewebe ausüben. Ein Nachteil der perfluorierten Alkane ist ferner ihr überdurchschnittlich hoher Preis und andererseits ihre extreme Hydrophobie und Lipophobie. Perfluorierte Alkane sind praktisch kaum löslich, weder in hydrophilen, noch lipophilen oder fluorophilen , Substanzen, was die Entfernung des Präparats aus dem Inneren des Glaskörperraumes enorm erschwert und zusätzliche Kosten verursacht.

Beim Einsatz von Vitrektomen, die mit unterschiedlichen Schnittraten arbeiten, kann ein weiteres Problem auftreten. Wenn sehr dicht an der Netzhaut gearbeitet wird, kann diese zu flattern beginnen, was es für den Chirurgen schwierig macht, genau zu arbeiten, und die Netzhaut gefährdet.

Aufgrund der Nachteile der bislang bekannten Spüllösungen für chirurgische Eingriffe und insbesondere beim Einsatz bei ophthalmologischen Eingriffen, besteht der Wunsch nach einem verbesserten Präparat.

Aufgabe der Erfindung war es nun, eine Spüllösung für bereitzustellen, die dem Chirurg die Durchführung des operativen Eingriffes erleichtert und dabei Sekundärverletzungen vorbeugt, die leicht verfügbar ist, die physiologisch annehmbar und gut verträglich ist und gegenüber den oben erwähnten perfluorierten Alkanen deutlich im Preis reduziert ist. Weiterhin war es Aufgabe der Erfindung eine Spüllösung für chirurgische Eingriffe am Auge bereitzustellen, die physiologisch verträglich und gut einführbar ist, gleichzeitig aber eine gute Sicht auf das Operationsfeld zulässt und die die Verwendung von Operationsgeräten, z.B. Vitrektomen erleichtert.

Diese Aufgabe wird gelöst durch eine Spüllösung zur Verwendung in einem Verfahren bei ophthalmologischen Eingriffen, insbesondere in der Glaskörper- und Netzhautchirurgie wie sie in Anspruch 1 definiert ist, umfassend Verwenden des Silikonöls bei dem Eingriff.

Es wurde überraschenderweise festgestellt, dass es für chirurgische Eingriffe vorteilhaft ist, eine nichtwässrige und nicht mit Wasser mischbare Nichtnewton'sche Flüssigkeit, die im vorliegenden Fall ein Silikonöl ist, als Spüllösung zu verwenden, da eine solche Spüllösung sich einerseits nicht mit Blut vermischt, wodurch eine Eintrübung und damit Beeinträchtigung der Sicht verhindert wird, und andererseits Scherkräfte, die bei Anwendung von chirurgischen Geräten entstehen, dämpfen kann. Dies ist generell bei chirurgischen Eingriffen vorteilhaft, vor allem dann, wenn Instrumente eingesetzt werden, deren Scherkräfte sich nachteilig auswirken.

Mit der erfindungsgemäßen Spüllösung wird erreicht, dass keine Vermischung mit den am Eingriffsort vorhandenen Flüssigkeiten, insbesondere Blut erfolgt, sodass dem Arzt eine bessere Übersicht ermöglicht wird. Außerdem wird ein Flattern oder Bewegen von Gewebe unter Einwirkung von Scherkräften verhindert oder vermindert. Letzteres ist besonders wichtig bei empfindlichen Geweben, wie der Netzhaut.

Wesentlich für die vorliegende Erfindung ist die Verwendung einer Nichtnewton' sehen Flüssigkeit, die im vorliegenden Fall ein Silikonöl ist, in einem Verfahren bei ophthalmologischen Eingriffen für das Spülen während eines operativen Eingriffs. Nichtnewtons'sche Flüssigkeiten sind definiert als solche Flüssigkeiten, bei denen die Schubspannung bei konstanter Temperatur dem Geschwindigkeitsgradienten nicht proportional ist bzw. die auf sie einwirkende Scherkräfte aufnehmen und damit dämpfen.

Die eingesetzte erfindungsgemäße Spülflüssigkeit ist darüber hinaus nicht oder kaum mit Wasser mischbar, um die Vermischung mit Blut und damit die Anfärbung zu vermeiden. Unter einer nicht oder kaum mit Wasser mischbaren Flüssigkeit wird ein in Wasser kaum, schwer löslich oder sehr schwer lösliches Lösungsmittel verstanden, das nur in einem Anteil von weniger als 20 Teilen pro 100 Teilen Wasser löslich ist, bevorzugt weniger als 10 Teilen, besonders bevorzugt weniger als 5 Teilen.

Für die Verwendung in der Ophthalmologie ist während des Eingriffs eine Unterscheidurig zwischen Glaskörper und Spüllösung notwendig, was die erfindungsgemäße Spüllösung ermöglicht. Gleichzeitig sollte zu jeder Zeit die Glaskörperbasis gut erkennbar sein, um den Eingriff für den Chirurgen zu erleichtern, insbesondere um sekundäre Verletzungen durch die chirurgische Prozedur zu vermeiden.

Überraschender Weise wurde gefunden, dass Silikonöle mit einer spezifischen Dichte von weniger als 1 g/cm³ und einer spezifischen Viskosität von nicht mehr als 100 mPas, gemessen bei einer Temperatur von 25°C bei Umgebungsdruck mit einem Ubbelohde-Kapillarviskosimeter, sich als Spüllösung für ophthalmologische Eingriffe besonders gut eignen und nicht nur die teueren perfluorierten Verbindungen ersetzen können, sondern darüber hinaus noch weitere Vorteile bieten.

Silikonöle sind seit langem bekannt. Sie finden in vielen Bereichen Anwendung, z.B. in industriellem Bereich als Entschäumungsmittel oder für Beschichtungen, aber auch in der Kosmetik, z.B. als Glanzgeber und die Haftung verbesserndes Mittel. In der Medizintechnik sind sie überwiegend aus der plastischen Chirurgie bekannt.

Durch mannigfaltige Synthesen lassen sie sich je nach Anwendungsgebiet in beliebiger Konstitution synthetisieren. Sowohl zyklische als auch aliphatische Silikone und insbesondere Siloxane und darunter Polydimethylsiloxane (PDMS) sind hierunter zu finden. Dabei reicht die Spannweite der aliphatischen Silikone und insbesondere Siloxane von niedrigmolekular bis hochmolekular, wobei sich unter den hochmolekularen solche mit Viskositäten von weit über 1 Million mPas befinden.

Verfahren zur Herstellung von Silikonölen sind bekannt und Silikonöle sind gut und zu relativ günstigen Preisen erhältlich.

Die Erfinder haben nun überraschend gefunden, dass sich die erfindungsgemäßen spezifischen Silikonöle sehr gut zur Verwendung in einem Verfahren bei ophthalmologischen Eingriffen als Spüllösung eignen.

Ein wichtiger Vorteil der verwendeten Nichtnewton'schen Flüssigkeiten, die erfindungsgemäß Silikonöle sind, ist ihre schlechte Vermischbarkeit mit Blut. Die bisher verwendeten Spüllösungen vermischen sich mit Blut, was zu Sichtbehinderungen bei der Operation führt. Mit Silikonen findet keine Vermischung statt, sondern es bildet sich eine Phasengrenze, die für den Arzt gut sichtbar ist. Außerdem kann das Silikonöl das Blut zurückdrängen, sodass unter Umständen sogar eine gewisse blutstillende Wirkung auftritt.

Die bleibende Transparenz der verwendeten Nichtnewton'schen Flüssigkeiten, die erfindungsgemäße Silikonöle sind, die nicht durch Blut oder andere wässrige Lösungen beeinträchtigt wird, hat noch einen weiteren Vorteil. Die erfindungsgemäße Spüllösung ist bevorzugt dazu vorgesehen, unter anderem bei der Vitrektomie als Spüllösung eingesetzt zu werden. Die Vitrektomie wird durchgeführt, um den Glaskörper zu entfernen, was notwendig ist, wenn der Glaskörper entzünden ist, wenn ein Eingriff an der Netzhaut vorzunehmen ist oder wenn der Glaskörper undurchsichtig geworden ist. In jedem Fall muss der Glaskörper so entfernt werden, dass die Netzhaut nicht mit abgezogen wird. Der Glaskörper liegt an der Netzhaut über die Glaskörpergrenzmembran an. Wenn daher der Glaskörper bereits eingetrübt ist, ist es umso wichtiger, dass die Spüllösung weder trüb ist noch durch Blut und andere Bestandteile eingetrübt wird.

Eine wichtige Eigenschaft des erfindungsgemäß eingesetzten Silikonöls ist die Dichte. Nur solche Silikonöle, die eine Dichte kleiner als die von Wasser, also kleiner 1,0 g/cm³ aufweisen, sind geeignet. Bevorzugt werden Silikonöle mit einer Dichte im Bereich von 0,80 bis 0,98 g/cm³, besonders bevorzugt 0,90 bis 0,98 g/cm³ eingesetzt. Aufgrund der gegenüber Wasser geringeren Dichte sind derartige Silikonöle in der Lage, auch den oberen Bereich des Glaskörperraumes auszufüllen. Durch Infusion des erfindungsgemäßen Silikonölpräparats wird die subretinale Flüssigkeit in den hinteren Bereich des Glaskörperraumes verdrängt. Dies birgt entscheidende Vorteile, insbesondere bei Operationen im vorderen Bereich des Glaskörperraumes. Dort dient die erfindungsgemäße Infusionslösung nicht nur als Volumenersatz für den Glaskörper, sondern fungiert auch als Netzhautstabilisator, der ein Kollabieren derselben verhindert.

Ein anderer Vorteil der erfindungsgemäßen Spüllösung ist auf ihre nichtnewton'schen Eigenschaften zurückzuführen. Es wurde gefunden, dass die erfindungsgemäße Spüllösung die Schwingungen oder das Flattern der Netzhaut dämpfen kann, die bei Einsatz eines Vitrektoms nahe an der Netzhaut entstehen. Das Silikonöl trägt auch aus diesem Grund dazu bei, den Eingriff für den Chirurgen besser kontrollierbar und sicherer zu machen, und damit zu erleichtern.

Die zweite wichtige Eigenschaft des erfindungsgemäßen Silikonöls ist die Viskosität. Nur wenn die Viskosität ausreichend klein ist, kann die erfindungsgemäße Spüllösung über dünne Kanülen oder Nadeln zugeführt und abgesaugt werden. Die Viskosität darf daher einen Wert von 100 mPas nicht übersteigen, wenn bei 25°C und Umgebungsdruck mit einem UBBELOHDE-Kapillarviskosimeter Schott CT52 gemessen wird.

Die erfindungsgemäßen Silikonöle mit einer Viskosität von etwa 0,1 bis 100 mPas zeichnen sich gegenüber den im Stand der Technik verwendeten perfluorierten Alkanen durch eine deutlich geringere Viskosität aus. Es wurde gefunden, dass Silikonöle, die eine Viskosität in diesem Bereich aufweisen, gut zu verarbeiten sind. Sie lassen sich gut durch dünne Kanülen, wie sie bei den entsprechenden chirurgischen Eingriffen verwendet werden, in das Innere des Auges überführen und auch wieder daraus absaugen. Bei Eingriffen am Auge kommen mittlerweile Kanülen mit bis zu 25 Gauge zum Einsatz, durch deren sehr enge Lumina die Flüssigkeit fließen muss. Die erfindungsgemäße Spüllösung ist auch durch diese Kanülen applizierbar. Silikonöle, die eine höhere Viskosität als 100 mPas aufweisen sind zähflüssig und können durch derart dünne Kanülen gar nicht und durch dicke Kanülen nur begrenzt zugeführt und abgesaugt werden. Niedrigviskosere Silikonöle, beispielsweise mit einer Viskosität von 0,1 mPas sind zwar extrem dünnflüssig, jedoch ist ihr Dampfdruck und damit verbunden ihre Flüchtigkeit zu hoch, um noch sinnvoll eingesetzt werden zu können. Darüber hinaus kann es bei Silikonölen mit einer Viskosität unter 0,1 mPas Probleme bei der Sterilisierbarkeit geben. Auch könnten Silikone mit nur 1 Siloxaneinheit aus Gründen der Toxizität für eine am Auge anzuwendende Lösung ungeeignet sein, was bei den erfindungsgemäß definierten nicht der Fall ist. Im Gegenteil zeichnen sich die erfindungsgemäß in Betracht gezogenen Silikonöle gerade durch ihre Biokompatibilität aus.

Besonders geeignet sind daher Silikonöle, deren Viskosität zwischen etwa 0,5 bis 50 mPas liegt und bevorzugter zwischen 1 und 20 mPas. In diesem Bereich ist ein ausgewogenes Verhältnis zwischen Flüchtigkeit und Viskosität gegeben, so dass derartige Silikonöle leicht durch entsprechende Kanülen in den Glaskörperraum einzuführen sind und ihre Flüchtigkeit so gering ist, dass sie in diesem verbleiben ohne sich durch die anderen chirurgischen Einschnitte oder über die Hornhaut zu verflüchtigen. Besonders gut eignen sich Silikonöle, deren Viskosität zwischen etwa 2 und 10 mPas liegt. Dieses weisen demgegenüber noch einmal eine deutlich erniedrigte Flüchtigkeit auf als Silikone mit einer Viskosität kleiner als 2 mPas. Die obere Grenze von 10 mPas gibt dabei den Wert an, der ein optimales Fließverhalten des Silikonöls und daher die Herstellung einer besonders gut geeigneten Infusionlösung für ophthalmologische Eingriffe zulässt.

Als Silikonöl werden erfindungsgemäß fließfähige Silikone, insbesonderee Siloxane eingesetzt, die die beiden geforderten Eigenschaften erfüllen. Gut geeignet sind Methylsiloxane und deren Polymere und Dimethylsiloxane und deren Polymere Die Silikone können sowohl unsubstituiert als auch substituiert vorliegen. Unter Substitution wird dabei der Ersatz eines an ein Siliciumatom gebundenes Wasserstoffatom durch ein anderes Atom oder eine Molekülgruppe verstanden. Gängige Substituenten sind dabei geradkettige, verzweigte oder cyclische Alkylgruppen mit 1 bis 18 Kohlenstoffatomen oder Arylgruppen mit 6 bis 15 Kohlenstoffatomen. Die Alkyl- bzw. Arylgruppen können dabei selbst wieder substituiert sein. Die Substituenten können blockweise oder verteilt über die Siloxankette vorliegen und ihr Anteil kann in an sich bekannter Weise nach den gewünschten Eigenschaften eingestellt werden.

Erfindungsgemäß kommen insbesondere unsubstituierte sowie alkyl- als auch arylsubstituierte Silikonöle in Betracht. Besonders eignen sich Polydimethylsiloxane (PDMS), die unsubstituiert oder aber substituiert sein können. Im Falle von substituierten Silikonen sind aryl- und darunter phenylsubstituierte Produkte gut verfügbar.

Silikonöle im Allgemeinen kennzeichnet eine Unmischbarkeit mit Wasser und daher auch mit Blut. Aufgrund dessen bildet sich nach dem Einführen des erfindungsgemäßen Präparats in den Glaskörperraum des Auges an der Phasengrenze zwischen Silikonöl und Glaskörper eine Grenzfläche aus. Die unterschiedlichen Brechungsindizes von Silikonöl und Wasser haben daher den Vorteil, dass der Chirurg zu jedem Zeitpunkt der Operation weiß, wo er sich mit seinem chirurgischen Instrument befindet, ob im Glaskörper oder im Silikonöl, und er kann besser beurteilen, ob der Glaskörper vollständig entfernt wurde. Die Ausbildung der Grenzfläche hilft dem Chirurgen bei der Orientierung. Unter diesem Aspekt geeignet sind daher solche Silikonöle, deren Brechungsindex sich von dem von Wasser deutlich unterscheidet, Von den substituierten Silikonölen sind in dieser Hinsicht insbesondere phenyl-substituierte Silikonöle vorteilhaft, wie Phenyldimethicone, Phenylmethicone, Phenyltrimethicone, Diphenyldimethicone, Diphenylmethicone, Diphenyltrimethicone, da Phenylgruppen den Brechungsindex erhöhen und darüber hinaus für eine erhöhte Transparenz sorgen. Der erhöhte Brechungsindex macht die Spanne zwischen den Brechungsindizes von Wasser und Silikonöl, noch größer, so dass die Grenzfläche der beiden Flüssigkeiten für den Chirurg noch deutlicher in Erscheinung tritt. Dies führt zu einer erheblichen Vereinfachung des komplizierten chirurgischen Eingriffes und damit zu einem verbesserten Operationsergebnis.

Unter den Silikonölen, die für die erfindungsgemäße Spüllösung geeignet sind, finden sich unter anderem: n-Polydimethylsiloxane, iso-Polydimethylsiloxane, arylsubstituierte PDMS, wie Phenyl-Polydimethylsiloxane, Diphenyl-Polydimethylsiloxane, Polyphenyl-Polydimethylsiloxane, alkylsubstituierte PDMS, wie Methyl-Polydimethylsiloxane, Ethyl-Polydimethylsiloxane, Propyl-Polydimethylsiloxane und andere, anderweitig substituierte PDMS, wie Fluor-alkyl substituierte PDMS. Diese Aufzählung soll beispielhaft geeignete Silikonöle für den Einsatz in einer erfindungsgemäßen Spüllösung wiedergeben, jedoch nicht auf sie beschränkt sein. Im folgenden sind einige geeignete Verbindungen beispielhaft angeführt: Bis-Diphenylethyl Disiloxane, Bisphenylhexamethicone, Capryl Dimethicone, Caprylyl Dimethicone, Caprylyl Methicone, Dimethicone, Disiloxane, Hexyl Dimethicone, Hexyl Methicone, Lauryl Dimethicone, Lauryl Methicone, Methicone, Methyl Trimethicone, Phenylethyl Dimethicone, Phenylethyl Disiloxane, Phenylpropyl Ethyl Methicone, Phenyl Propyl Trimethicone, Phenyl Trimethicone, Trifluoropropyl Dimethicone, Trifluoropropyl Methicone, Trisiloxane u.a..

Wie oben ausgeführt werden erfindungsgemäß Silikonöle verwendet, um die mit der Verwendung von perfluorierten Alkanen verbundenen Nachteile auszuräumen. Auch die Verwendung von semifluorierten Alkanen ist aus ähnlichen Gründen nicht bevorzugt. Wegen der guten Mischbarkeit von semifluorierten Verbindungen und Silikonen kann allerdings das Zumischen eines geringen Anteils an semifluorierten Verbindungen in manchen Fällen nützlich sein, z.B. um die Dichte und/oder Viskosität gezielt einzustellen. Bevorzugt können, abhängig von den verwendeten Stoffen und abhängig von dem vorgesehen Zweck, bis zu 10 Vol.-% semifluorierte Alkane zugemischt werden. Geeignete semifluorierte Alkane werden beispielsweise in EP0859751 beschrieben.

Der Zusatz eines semifluorierten Alkans bietet einen weiteren Vorteil. Semifluorierte Alkane besitzen eine höhere Lösungsfähigkeit für Wirkstoffe als Silikone. In einer bevorzugten Ausführungsform können daher der erfindungsgemäßen Spüllösung ein oder mehrere semifluorierte Alkane als Lösungsvermittler für Wirkstoffe zugesetzt werden. Als Wirkstoffe in diesem Zusammenhang kommen Medikamente, Antioxidantien, Nährstoffe für das Auge oder ähnliches in Betracht. Als Beispiele für Medikamente können lindernde oder antibiotische Mittel genannt werden. Beispiele für Antioxidantien sind Verbindungen, die freie Radikale abfangen. Als Nährstoffe sind Zucker, wie Glucose, zu nennen.

Der Anteil des oder der semifluorierten Alkane in der Spüllösung, bevorzugt in Silikon kann variieren abhängig von den gewünschten Eigenschaften. Mit dem semifluorierten Alkan lässt sich nicht nur die Löslichkeit für bestimmte Mittel verbessern, sondern auch die Dichte einstellen. Bevorzugt wird semifluoriertes Alkan in einem Anteil von mehr als 1 Gew.-%, bevorzugter mehr als 5 Gew.-%, zugefügt. Der Anteil an semifluoriertem Alkan kann auch höher sein. Der Fachmann kann das geeignete Verhältnis von Slikon zu semifluoriertem Alkan leicht mit wenigen Routineversuchen einstellen, wobei ein Verhältnis von Slikon zu semifluoriertem Alkan im Bereich von 50:1 bis 1 :2 besonders geeignet ist.

Die beschriebenen Silikonöle eignen sich für die Herstellung von Spüllösungen für ophthalmologische Eingriffe, insbesondere auch als intraoperatives Instrument, insbesondere als Instrument zur Manipulation von intraokularen Strukturen. Die erfindungsgemäßen Präparate sind bei ophthalmologischen Operationen insbesondere zum Spülen des Glaskörperraumes vorteilhaft. Die erfindungsgemäßen Präparate werden daher in Form einer Spüllösung bereitgestellt, die bei ophthalmologischen Eingriffen, insbesondere im Netzhautbereich, zur Anwendung kommen

## Patentansprüche

1. Silikonöl mit einer Dichte von weniger als 1 g/cm³ und einer Viskosität von nicht mehr als 100 mPas, gemessen bei 25°C und Umgebungsdruck mit einem Ubbelohde-Kapillarviskosimeter, zur Verwendung in einem Verfahren bei ophthalmologischen Eingriffen, umfassend Verwenden des Silikonöls als Spüllösung bei dem ophthalmologischen Eingriff.

2. Silikonöl zur Verwendung nach Anspruch 1, wobei die Spüllösung bei der Glaskörper- und Netzhautchirurgie, bei der Vitrektomie, im hinteren Bereich des Auges oder zum Zurückdrängen von Blut verwendet wird.

3. Silikonöl zur Verwendung nach Anspruch 2, **dadurch gekennzeichnet, dass** es eine Dichte von 0,80 bis 0,98 g/cm³ hat.

4. Silikonöl zur Verwendung nach einem der vorhergehenden Ansprüche **dadurch gekennzeichnet, dass** sie eine Viskosität in einem Bereich von 0,5 bis 50 mPas hat

5. Silikonöl zur Verwendung nach einem der vorhergehenden Ansprüche **dadurch gekennzeichnet, dass** sie eine Viskosität in einem Bereich von 2 bis 10 mPas hat

6. Silkonöl zur Verwendung nach einem der vorhergehenden Ansprüche, **dadurch gekennzeichnet, dass** das Silikonöl ein geradkettiges Siloxan ist, und/oder **dadurch gekennzeichnet, dass** das Silikonöl ein substituiertes oder unsubstituiertes Polydimethylsiloxan ist, und/oder **dadurch gekennzeichnet, dass** das Silikonöl aus Siloxanen mit 2 bis 10 Siloxaneinheiten gebildet wird.

7. Silikonöl zur Verwendung nach einem der vorhergehenden Ansprüche **dadurch gekennzeichnet, dass** das Silikonöl phenylsubstituiert ist.

8. Silikonöl zur Verwendung nach einem der vorhergehenden Ansprüche **dadurch gekennzeichnet, dass** zusätzlich ein semifluoriertes Alkan enthalten ist.

9. Silikonöl zur Verwendung nach Anspruch 7, **dadurch gekennzeichnet, dass** Silikonöl und semifluoriertes Alkan in einem solchen Verhältnis enthalten sind, dass die Dichte der Lösung kleiner als 1 g/cm³ ist.

10. Silikonöl zur Verwendung nach Anspruch 7 oder 8, **dadurch gekennzeichnet, dass** Silikonöl und semifluoriertes Alkan in einem Verhältnis von 50:1 bis 1:2 enthalten sind.

11. Silikonöl zur Verwendung nach einem der vorhergehenden Ansprüche **dadurch gekennzeichnet, dass** zusätzlich ein oder mehrere Wirkstoffe, bevorzugt ein oder mehrere wasserunlösliche Wirkstoffe enthalten sind.

12. Silikonöl zur Verwendung nach Anspruch 5, **dadurch gekennzeichnet, dass** ein oder mehrere Medikamente, Antioxidantien und/oder Nährstoffe enthalten sind.

## Claims

1. Silicone oil having a density of less than 1 g/cm³ and a viscosity of not higher than 100 mPas, measured at 25°C and ambient pressure with a Ubbelohde capillary viscometer, for use in a method in ophthalmic surgeries, comprising using the silicone oil as a flushing solution in said ophthalmic surgery.

2. Silicone oil for use according to claim 1, wherein the flushing solution is used in the vitreous body and retinal surgery, during vitrectomy, in the posterior region of the eye, or for pushing back the blood.

3. Silicone oil for use according to claim 2, **characterized in that** it has a density from 0.80 to 0.98 g/cm³.

4. Silicone oil for use according to any one of the preceding claims, **characterized in that** it has a viscosity in the range from 0.5 to 50 mPas.

5. Silicone oil for use according to any ane of the preceding claims, **characterized in that** it has a viscosity in the range from 2 to 10 mPas.

6. Silicone oil for use according to any one of the preceding claims, **characterized in that** the silicone oil is a straight-chained siloxane, and/or **characterized in that** the silicone oil is a substituted or unsubstituted polydimethylsiloxane, and/or **characterized in that** the silicone oil is formed from siloxanes having from 2 to 10 siloxane units.

7. Silicone oil for use according to any ane of the preceding claims, **characterized in that** the silicone oil is phenyl-substituted.

8. Silicone oil for use according to any one of the preceding claims, **characterized in that** a semi-fluorinated alkane is additionally present.

9. Silicone oil for use according to claim 7, **characterized in that** the silicone oil and the semi-fluorinated alkane are present in a ratio such that the density of the solution is less than 1 g/cm³.

10. Silicone oil for use according to claim 7 or 8, **characterized in that** the silicone oil and semi-fluorinated alkane are present in a ratio of from 50:1 to 1:2.

11. Silicone oil for use according to any ane of the preceding claims, **characterized in that** one or more active ingredients, preferably one or more water-insoluble active ingredients, are additionally present.

12. Silicone oil for use according to claim 5, **characterized in that** one or more medicaments, antioxidants, and/or nutrients are present.

## Revendications

1. Huile silicone ayant une densité inférieure à 1 g/cm³ et une viscosité non pas supérieure à 100 mPas, mesurée à 25 °C et à pression ambiante à l'aide d'un viscosimètre capillaire Ubbelohde, pour être utilisée dans un procédé lors d'interventions ophtalmologiques, comprenant l'utilisation de ladite huile silicone en tant que solution de rinçage lors de l'intervention ophtalmologique.

2. Huile silicone pour l'utilisation selon la revendication 1, dans laquelle la solution de rinçage est utilisée dans la chirurgie du corps vitré et de la rétine, lors de la vitrectomie, dans la zone arrière de l'oeil ou pour refouler le sang.

3. Huile silicone pour l'utilisation selon la revendication 2, **caractérisée par le fait qu'**elle présente une densité comprise entre 0,80 et 0,98 g/cm³.

4. Huile silicone pour l'utilisation selon l'une quelconque des revendications précédentes, **caractérisée par le fait qu'**elle présente une viscosité se situant dans une plage allant de 0,5 à 50 mPas.

5. Huile silicone pour l'utilisation selon l'une quelconque des revendications précédentes, **caractérisée par le fait qu'**elle présente une viscosité se situant dans une plage allant de 2 à 10 mPas.

6. Huile silicone pour l'utilisation selon l'une quelconque des revendications précédentes, **caractérisée par le fait que** l'huile silicone est un siloxane à chaîne droite, et/ou **caractérisée par le fait que** l'huile silicone est un polydiméthylsiloxane substitué ou non substitué, et/ou **caractérisée par le fait que** l'huile silicone est formé de siloxanes ayant entre 2 et 10 unités de siloxane.

7. Huile silicone pour l'utilisation selon l'une quelconque des revendications précédentes, **caractérisée par le fait que** l'huile silicone est substituée par du phényle.

8. Huile silicone pour l'utilisation selon l'une quelconque des revendications précédentes, **caractérisée par le fait que**, en sus, un alcane semi-fluoré est contenu.

9. Huile silicone pour l'utilisation selon la revendication 7, **caractérisée par le fait que** l'huile silicone et l'alcane semi-fluoré sont contenus dans un rapport tel que la densité de la solution est inférieure à 1 g/cm³.

10. Huile silicone pour l'utilisation selon la revendication 7 ou 8, **caractérisée par le fait que** l'huile silicone et l'alcane semi-fluoré sont contenus dans un rapport allant de 50:1à1:2.

11. Huile silicone pour l'utilisation selon l'une quelconque des revendications précédentes, **caractérisée par le fait que**, en sus, un ou plusieurs principes actifs, de préférence un ou plusieurs principes actifs insolubles dans l'eau sont contenus.

12. Huile silicone pour l'utilisation selon la revendication 5, **caractérisée par le fait qu'**un ou plusieurs médicaments, antioxydants et/ou substances nutritives sont contenus.
